# EUROPEAN PATENT SPECIFICATION

(11) **EP 2 315 562 B1**
(45) Date of publication and mention of the grant of the patent: **14.08.2013**
(21) Application number: 09746092.7
(22) Date of filing: 08.05.2009
(51) Int. Cl.: A61F 9/007, A61M 3/02, A61B 19/00

(54) **OPHTHALMIC IRRIGATOR**
AUGENIRRIGATOR
IRRIGATEUR OPHTALMIQUE

(30) Priority: 10.05.2008 GB 0808509; 04.12.2008 GB 0822101
(43) Date of publication of application: 04.05.2011
(73) Proprietor: LJT Projects Limited, Maidenhead, Berkshire SL6 6TB (GB)
(72) Inventor: Pearson, Andrew Robert, Wargrave Berkshire, RG10 8PB (GB)
(74) Representative: Mansfield, Peter Turquand
(86) International application number: PCT/GB2009/050484
(87) International publication number: WO 2009/138775

(56) References cited:
- WO-A-93/08742
- US-A- 5 814 030
- US-A- 6 030 356

## Description

The present invention relates to an improved device for use by an ophthalmic surgeon during cataract or other intra-ocular surgery.

During such eye surgery the surgeon needs to have a clear view down a microscope of what he is doing with his instruments inside the eye. Typically, the patient lies supine and looks vertically upwards towards the microscope. The cornea is exposed by a clip or speculum that retracts the eyelids thus preventing the normal blinking mechanism from keeping the cornea moist. Drying of the cornea during surgery converts a uniformly transparent cornea to a less than regular surface that impairs the view of the tissues within the eye.

To counter this the surgeon's assistant typically has a small bottle of suitable fluid, usually appropriate saline or 'balanced salt solution' attached to a cannula which is used to intermittently irrigate the cornea to restore an even surface and a clear view. The surgeon is unable to do this because both hands are already in use with the eye surgery equipment, so is dependant on the assistant providing the right amount of irrigation at the right place at the right time.

This may not always occur for numerous reasons, for example the assistant may have a temporary lack of concentration, or lack of an adequate view of the eye, or be performing some other task, and so forth. Alternatively fluid may be dispensed when it is not desired. Such fluid momentarily impairs the surgeon's view and, if this occurs at an important point in the operation, the surgery may be compromised.

The frequency of irrigation needed is also variable depending on several factors that alter the rate at which the cornea dries out such as the type of anaesthetic used, the amount of antiseptic used, pre-existing abnormalities of the surface of the patient's eye, the warmth/humidity of the operating theatre, and so on. Each patient and operation is different.

Prior to the current invention it was therefore known to use the help of an assistant to irrigate the eye by using a simple bottle.

A device known as a 'Drews microscope dripper' is also known. It has a steel ring that can be screwed on to a microscope objective lens, attached to the ring being a flexible attachment mechanism holding a cannula for directing irrigating fluid to the cornea under an assistant's control. Other proposed designs are described in the following patent publications:
JP-A-2003-250832 describes an instillator attachment for use with an ophthalmic microscope having a pipe portion attached to the microscope, one end of the pipe portion extending toward the operation field in a manner that can be varied manually.
US 5,814,030 describes a corneal irrigation device that can be attached to a microscope. The device directs the flow of irrigation fluid discharged by a cannula that is mounted on a manually controllable ball and socket joint attached to a bracket, which is itself connectable to the microscope.
US 6,179,829 and US-A-4,790,816 describe foot control assemblies for controlling a plurality of ophthalmic instruments on microsurgical systems.
US-A-5,562,612 describes an apparatus for ophthalmic irrigation that uses a solenoid valve for controlling the rate of flow of irrigation fluid.

Prior to the present invention, a simple microscope-mounted system where the process of set-up ensures that irrigation fluid is automatically directed in a predetermined way to a required corneal location without requiring surgeon or assistant manual adjustment, that can maintain its position relative to the eye during an operation, and where the release of fluid is controllable by the surgeon, such features ensuring that the eye's surface is kept in an ideal state of hydration throughout the surgery, had not been provided.

It is one aim of the present invention to provide such a simple, easy to use apparatus.

Thus, according to one aspect, the present invention provides an ophthalmic irrigation device as claimed in accompanying claim 1.

Hence, a microscope-mountable irrigation device is provided that can supply fluid through an irrigator tip whose position with respect to the eye is predetermined to deliver the fluid at the correct location. This location would normally be the centre of the viewing field of the microscope when the object in view at this point is in focus. The flow of fluid can be controlled by the surgeon using a foot-pedal controller unit that may also be used to control the position of the objective lens and so forth of the microscope.

An important feature of the invention is the provision of its microscope-mountable bracket. The arrangement of having an irrigator tip attached by this bracket to the microscope provides the significant advantage that it can ensure that the tip is always at the correct position relative to the eye. The ability to predetermine the location of the fluid supply depends upon the relationship between the known dimensions of the bracket and those of the tube's tip and the fixings that hold it to the bracket, as well as the trajectory of fluid flow. A preset height to the bottle of fluid is used so that the irrigation fluid automatically comes out of the irrigation tip at a predetermined rate. The required trajectory of the irrigation fluid is such that the fluid falls on the cornea when the cornea is in focus in the centre of the viewing field, and this determines the required position of the irrigation tip. By choosing appropriate dimensions for the bracket and the tube's fixings the location of the tip of the tube relative to the objective lens of the microscope can be predetermined to be in this position and is automatically the same each time the system is used, without any manual adjustment being needed.

During surgery, as a result of patient movement during the operation (or the need to view an adjacent part of the eye), the microscope itself is constantly: (a) being realigned with the eye in the X-Y (horizontal) plane to ensure the eye is in the middle of the viewing area down the microscope, and (b) also being moved up and down vertically (the Z plane) relative to the eye to ensure that the eye remains in focus. Because the bracket and objective lens are attached to the same part of the body of the microscope, movements of the microscope, under the control of the surgeon during the operation, to keep the eye in the centre of the viewing field cause a corresponding movement of the tip of the irrigating tube. The irrigator will therefore reliably and accurately maintain a correct position relative to the eye throughout the operation even if the patient has moved on the couch, and continue to supply fluid at the correct location. In this way, the surgeon's view is optimised, which ultimately leads to the associated surgery becoming more effective.

Further advantageous and optional features are mentioned in the accompanying subsidiary claims.

The present invention will now be described, in an otherwise non-limiting way, by way of the following preferred examples with reference to, and as illustrated in, the accompanying figures, in which:
Figure 1 schematically illustrates an example of a microscope having attached thereto an embodiment of the device of the present invention;
Figure 2 shows alternative positions for locating a saline bag as part of the fluid column required for such a microscope;
Figure 3 shows one embodiment of a connector, forming a part of the device of the present invention for attaching tubing to a microscope;
Figures 4a and 4b show an alternative to the connector of figure 3, at successive stages during assembly of the device;
Figure 4c shows another alternative to the connector of figure 3;
Figures 5a to 5i show alternative ways of connecting tubing to a connector as shown in figures 4b or 4c.

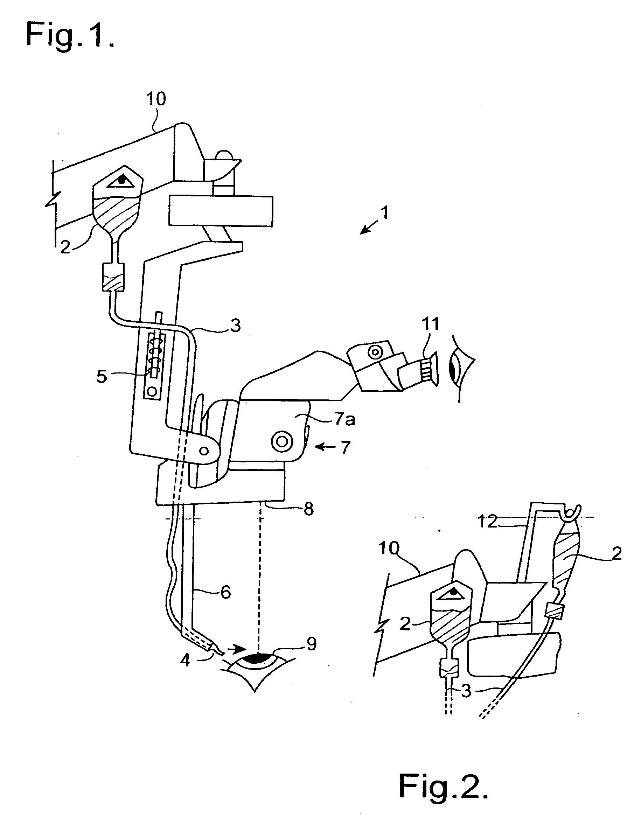

As shown in Figure 1, the present invention provides an irrigation device 1 that can be mounted upon a microscope 7 to provide irrigation in the field of view of the microscope. The device 1 consists of at least a bag 2 of irrigation fluid connected via a flexible tube 3 to an irrigation delivery tip 4. The flow of fluid through the tube 3 is controlled by a valve 5 that is operated by a remote controller unit (not shown) that is preferably a foot-pedal controller that is also used to control operation of the microscope 7. The tip 4 is held in place by a bracket 6 that is mounted upon the same part of the body 7a of the microscope 7 as the objective lens 8 that is used to view the patient's eye 9, so that the objective lens 8 and the bracket 6 always move together.

The microscope 7 is typically supported above the patient by an arm 10. The surgeon can view the patient's eye 9 by looking through eyepiece lenses 11, and can control the operation of both the valve 5 and microscope 7 via the remote controller unit, without needing assistance.

The bag 2 of fluid that is appropriate for irrigation of an eye typically contains 50-100 ml of 'normal' saline or balanced salt solution. The bag 2 may be mounted on the supporting arm 10 or, alternatively upon an optional mounting post 12 (see Figure 2) attached to the microscope 7 at the required height to provide sufficient flow down a gravity feed system to appropriately wet the cornea of the patient's eye 9 when flow is allowed. However, instead of relying upon gravity, a pressurised flow using a pump (not shown) can be used to supply the irrigation fluid.

The tube 3 connecting the bag 2 to the delivery tip 4 is typically attached to the microscope 7 at appropriate positions along its course that are chosen to keep it away from other relevant parts of the microscope.

A controller valve 5 may be located at a point along the tube 3 and, preferably, is attached relatively far away from microscope body 7a and, when activated is typically designed so as to minimise shake or movement of the microscope 7 itself. Optionally, the valve 5 may be a solenoid-type valve the control of which is by the foot-pedal controller unit (not shown). This type of valve 5 is effective, but any type of alternative valve that achieves the same degree of control can be used.

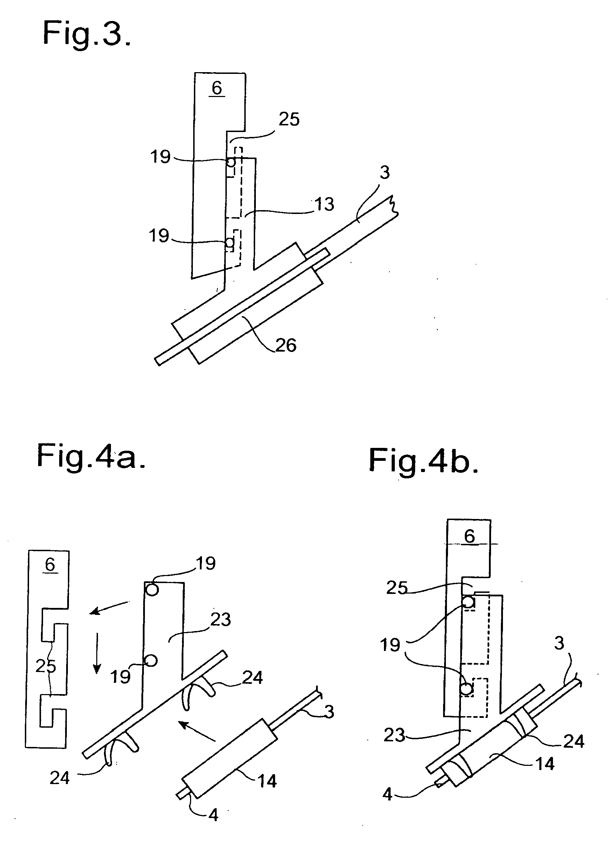

The bracket 6 (as shown by way of example in Figures 1 & 3) or some other equivalent type of delivery tip supporting means is used to attach the delivery tip 4 directly to the microscope 7. The bracket 6 is generally required to extend beyond the objective lens 8 of the microscope 7 towards the patient's eye 9. The vertical distance between the lens 8 and the eye 9 is usually approximately 15-25 cm and the bracket 6 typically brings the tip 4 of the irrigation device 1 to within about 2-5 cm of the eye 9, and, in this example, slightly to one side of the eye 9, for example between 5 mm and 25 mm to the side.

Preferably, the bracket 6 extends from the side of the microscope body 7a that is opposite to the viewing eyepieces 11 and is made of strong rigid material, for example a metal. Such positioning opposite the viewing eyepieces 11 serves to minimise the consequent reduction in working space under the microscope 7.

Figure 3 shows a connector unit 13 according to a preferred embodiment. A cannula 26 is embedded in the connector unit 13 that can be fixed into place with respect to the bracket 6. The supply tube 3 can be attached to an end of the cannula 26 that protrudes from the connecter unit 13. By way of example the cannula 26 may be of internal diameter 1.2 mm and of total length 30 mm.

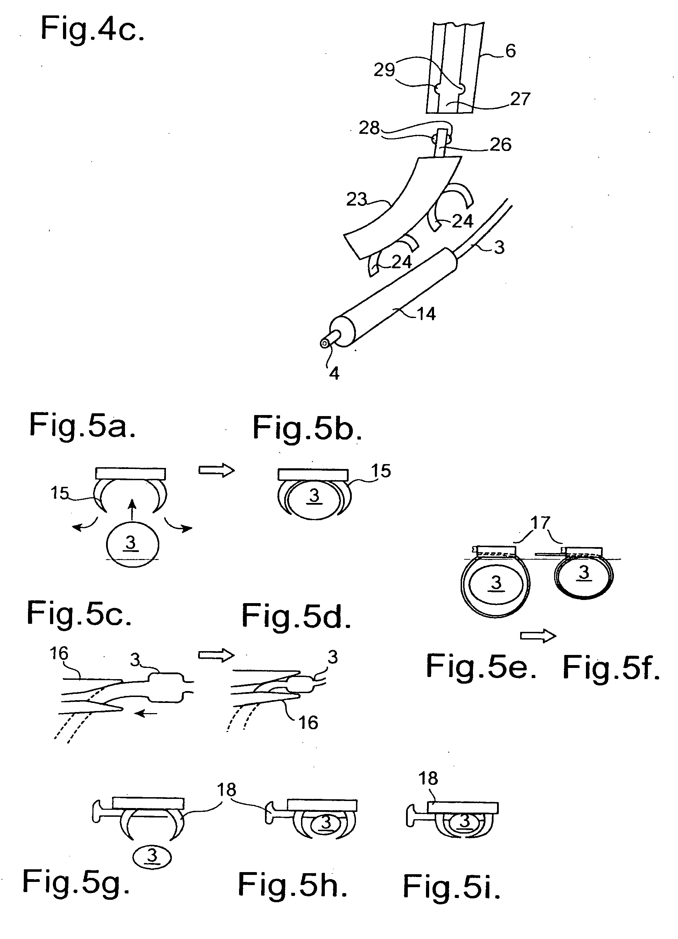

Alternatively, as shown in figure 4, a separate connector unit 23 can hold the end portion 14 of the tubing. Various different types of attachments 24 between tubing and connector unit may be used which are capable of holding the end portion 14 of the tubing in a predetermined, fixed position each time the connection is made; (some are discussed below in relation to figure 5). The connector unit 23 with tubing 3 attached is then mounted on the bracket 6 in a predetermined, fixed position.

As a non-illustrated alternative the end of the bracket 6 may instead include an attachment means 24 to hold the end of the tubing 3 in a predetermined, fixed position each time the connection is made with various different types of attachments (in an equivalent way to that shown with the separate connector unit 23).

As another non-illustrated alternative, a separate cannula (not shown) can be attached to the end of the tubing 3 or to the cannula 26 embedded in the connector unit 13.

A number of alternative arrangements for such clipping into a predetermined and fixed position of part of the tube 3 are shown in Figures 5a to i. Thus, a push-fit arrangement into spring clips 15 is shown in Figures 5a and b, a taper-fit between opposed jaws 16 is shown in Figures 5c and d, securing with a jubilee clip 17 is shown in Figures 5e and f, and a plunger-operated spring loaded clip 18 is shown in Figures 5g to il. In each case the end of the tube 3 is held in a fixed and predetermined position.

An advantage of having a separate connector unit 23 or a cannula/connector unit 13 lies firstly in the fact that it can be sterilised. The assistant can connect the sterile tubing end to the connector unit 23, or to the cannula 26 of the connector unit 13, and then mount it onto the non-sterile bracket 6 in much the same way as sterile caps are already placed over non-sterile microscope controls prior to surgery. Alternatively the connector unit 23 or cannula/connector unit 13 can be disposable and a new sterile one used for each operation.

A second advantage is that it allows adequate force to be applied to produce the necessary attachment between the end of the tubing and the connector unit 23 or the cannula/connector unit 13 without risk of bending or damaging the bracket 6. Also, the end of the tube 3 has a cap that needs to be removed and is often stiffly attached, so there is otherwise potential for the bracket 6 to be bent as removal of the cap is attempted. However, with a two piece arrangement using the connector unit 13 or 23 the cap can be removed in advance, thus minimising the risk of damage to the bracket 6.

Thirdly, different connector units 13 or 23 can be supplied to accommodate the different designs of irrigation tube end-portions 14 depending on regional operating theatre practice, since tubing end-portions 14 vary in configuration

Where a connector unit 23 or cannula/connector unit 13 is used, this is attached to the end of the bracket 6 in a predetermined position. As illustrated in figure 3, this can be achieved using downwardly extending grooves 25 in the bracket 6 into which bars 19 on the connector 13 fit. The same method of attachment is shown in figures 4a and 4b: in figure 4a the connector unit 23 includes two parallel bars 19 which are fitted into slots 25 near the bottom end of the bracket 6, so that as shown in figure 4b the connector unit 23 is firmly fixed to the bottom end of the bracket 6. In this way the connector 13 or 23 is effectively dropped into place on the bracket 6 and, in doing so, a downwards force can be applied that ensures that the bracket 6 is fully extended. Alternatively, as illustrated in figure 4c, a spring-loaded clip type attachment of the connector 13 or 23 is shown that can engage the bracket 6 securely if an assistant holds the bracket 6 (otherwise the bracket would be likely to slide up). For example in this case the connector 23 may include a cylindrical stub 26 that locates in a cylindrical bore 27 in the bottom end of the bracket 6, with spring-loaded balls or pins 28 protruding from the stub 26, that locate in corresponding recesses 29 within the cylindrical bore 27. However, any other similar system can be used that achieves the same result of providing a predetermined fixed relationship between the end tip 4 of the supply tube 3 and the bracket 6. As another example the pin 19 and slot 25 attachment mechanism of figures 3 and 4a and 4b might be modified by having only a single pin 19 and a single slot 25.

Optionally, the bracket 6 may be retractable so as to prevent damage when the microscope 7 is stored. This may be achieved by the whole bracket 6 being arranged to be slidable upwards out of the way of the patient into a more protected position for when the microscope 7 is being moved or in storage. Preferably, the bracket 6 is lockable in its retracted position, but hangs freely in the extended position with its full extension ensured by the effects of gravity working on the weight of the bracket 6 and the items attached to it, and the correct extension is typically ensured by a stop on the bracket 6. Hence, the risk of damage to what will be a relatively exposed and fragile item can be minimised. This also provides the added safety benefit of preventing or reducing damage to the patient in the event of their suddenly lifting their head during an operation.

In use, the device 1 is typically operated in the following manner. Prior to the start of the operation, a sterile assistant receives the sterile tubing 3 and, if used, the sterile connector unit or cannula/connector unit (s) 13. The cap (not shown) of the tubing 3 is removed. The sterile assistant then attaches the end-portion 14 of the tubing 3 directly to the bracket 6 or to a connector unit 23 or a cannula/connector unit 13, if used, and the connector unit 13 or 23 is then mounted on the bracket 6. The tubing 3 is given to a non-sterile assistant for attachment to the microscope 7 and connection to the bag 2 of fluid supported higher up on the microscope 7.

Alternatively, a non-sterile assistant can attach the end portion 14 of the tubing 3 to the end of the bracket 6 (or to the cannula/connector unit 13 or to the connector unit 23, as the case may be), the middle of the tubing to the microscope 7 and the top of the tubing to the fluid bag 2. The cap on the end of the tubing 3 can then be carefully removed to reveal the sterile tip 4 of the tube 3.

The microscope 7 is then brought into position via a gross movement control, usually activated by the surgeon pressing a release button on the microscope 7 that unlocks all the major hinge points and allows the microscope 7 to be swung into its position over the patient from its initially stored position away from the patient.

The button is then released, which locks the major points and residual fine control is achieved by way of foot pedal controls activating motors that move the microscope's body 7a (to which are attached the bracket 6 and the objective lens 8) so that the patient's eye 9 is in focus and in the centre of the viewing area.

A fluid control lever of the microscope controller is then actuated to allow liquid to run down the tubing 3 and irrigate the eye 9. As is evident from figure 1, the liquid is preferably projected onto the eye 9 from slightly above and to one side, so that the tip 4 of the tubing 3 does not obstruct the view of the surgeon. The liquid follows a parabolic trajectory onto the eye 9. Correct set up ensures the liquid is consistently applied to the correct position on the eye 9, at which or through which the microscope is focused. The microscope foot-pedal controller is used to control the frequency of irrigation during the operation. There is no adjustment of the tubing 3 or of the position of the tip 4 from which the liquid emerges, as these are predetermined by the length of the bracket 6 and the shape of the connector 13 or 23.

At the end of the operation the microscope 7 is removed and the tubing 3 disconnected. The connector unit 23 or cannula/connector unit 13, if used, can be disposed or sent for sterilisation as required; and the fluid bag 2 and tubing 3 discarded. The bracket 6 is retracted during microscope storage.

## Claims

1. An ophthalmic irrigation device (1) comprising:
a bracket (6) mountable upon a microscope (7) with a body (7a) and an objective lens (8), the bracket (6) being mountable onto the body (7a) such that the bracket (6) and the objective lens (8) are movable together; and are movable a fluid supply conduit (3) having an end tip (4) for supplying irrigation fluid to a patient's eye (9);
the bracket (6) having attachment means (13; 23, 24) arranged to receive the fluid supply conduit (3) and to provide a predetermined and fixed relationship of the end tip (4) to the bracket (6);
and the dimensions of the bracket (6), the attachment means (13; 23, 24), and the fluide supply conduit (3) being such that the end tip (4) in use is in a predetermined position relative to the objective lens (8) without any adjustment being needed, the predetermined position being that from which, in use, with the fluid supply conduit (3) in communication with a container (2) of irrigation fluid at a preset height, irrigation fluid falls at the centre of the viewing field of the microscope (7) when an object in view at this point is in focus.

2. A device as claimed in claim 1, wherein the fluid supply conduit (3) comprises a flexible supply tube.

3. A device as claimed in claim 2, wherein the fluid supply conduit (3) comprises a cannula/connector unit (26, 13), the cannula (26) arranged to provide the end tip (4) and the connector (13) arranged to receive and communicate with the irrigation fluid supply tube (3).

4. A device as claimed in any one of the preceding claims wherein the fluid supply conduit (3) is attached to the bracket (6) by a connector unit (23).

5. A device as claimed in claim 4 wherein the fluid supply conduit (3) is attached to the connector unit (23) by a releasable clip (24).

6. A device as claimed in claim 4 or claim 5, wherein the connector unit (23) comprises a spring loaded clip (28) that locates into a socket (27).

7. A device as claimed in claim 4 or claim 5, wherein the connector unit (23) comprises a bar (19) that locates into a slot (25).

8. A device as claimed in any one of the preceding claims wherein the bracket (6) is retractable, and hangs freely when in the extended position.

9. An ophthalmic microscope (7) comprising a device (1) as claimed in any one of claims 1 to 8.

10. A microscope as claimed in claim 9, comprising a container (2) of irrigation fluid arranged to supply irrigation fluid to the fluid supply conduit (3), the container being arranged at a predetermined height above the end tip (4) to gravity feed a desired flow of fluid from the end tip (4) to a patient's eye (9).

## Patentansprüche

1. Vorrichtung (1) zur Augenspülung, umfassend:
- eine Stütze (6), die an einem Mikroskop (7) mit einem Körper (7a) und einer Objektivlinse (8) montierbar ist, wobei die Stütze (6) derart an dem Körper (7a) montierbar ist, dass die Stütze (6) und die Objektivlinse (8) zusammen bewegbar sind; und
- eine Fluidzufuhrleitung (3), die eine Endspitze (4) aufweist, um einem Auge (9) eines Patienten Spülungsfluid zuzuführen;
wobei die Stütze (6) Befestigungsmittel (13; 23, 24) aufweist, die angeordnet sind, um die Fluidzufuhrleitung (3) aufzunehmen und um eine vorbestimmte und festgelegte Beziehung der Endspitze (4) zu der Stütze (6) bereitzustellen;
und wobei die Dimensionen der Stütze (6), der Befestigungsmittel (13; 23, 24) und der Fluidzufuhrleitung (3) derart sind, dass sich die Endspitze (4) im Gebrauch in einer vorbestimmten Position im Verhältnis zur Objektivlinse (8) befindet, ohne dass eine Anpassung notwendig wäre, wobei die vorbestimmte Position diejenige ist, von der aus im Gebrauch, wenn die Fluidzufuhrleitung (3) mit einem Behälter (2) des Spülungsfluids auf einer voreingestellten Höhe in Verbindung steht, das Spülungsfluid auf die Mitte des Blickfeldes des Mikroskops (7) fällt, wenn ein zu diesem Punkt sichtbare Objekt scharf eingestellt ist.

2. Vorrichtung nach Anspruch 1, wobei die Fluidzufuhrleitung (3) einen biegsamen Zufuhrschlauch umfasst.

3. Vorrichtung nach Anspruch 2, wobei die Fluidzufuhrleitung (3) eine Kanülen-/Verbinder-Einheit (26, 13) umfasst, wobei die Kanüle (26) angeordnet ist, um die Endspitze (4) bereitzustellen, und der Verbinder (13) angeordnet ist, um den Spülungsfluid-Zufuhrschlauch (3) aufzunehmen und damit in Verbindung zu stehen.

4. Vorrichtung nach einem der vorhergehenden Ansprüche, wobei die Fluidzufuhrleitung (3) durch eine Verbindungseinheit (23) an der Stütze (6) befestigt ist.

5. Vorrichtung nach Anspruch 4, wobei die Fluidzufuhrleitung (3) an der Verbindungseinheit (23) mit einer lösbaren Klammer (24) befestigt ist.

6. Vorrichtung nach Anspruch 4 oder 5, wobei die Verbindungseinheit (23) eine federbelastete Klammer (28) umfasst, die sich in einer Fassung (27) befindet.

7. Vorrichtung nach Anspruch 4 oder 5, wobei die Verbindungseinheit (23) einen Stab (19) umfasst, der in einen Schlitz (25) passt.

8. Vorrichtung nach einem der vorhergehenden Ansprüche, wobei die Stütze (6) einziehbar ist und frei hängt, wenn sie sich in der ausgezogenen Position befindet.

9. Ophthalmologisches Mikroskop (7), umfassend eine Vorrichtung (1) nach einem der Ansprüche 1 bis 8.

10. Mikroskop nach Anspruch 9, umfassend einen Behälter (2) mit Spülungsfluid, der angeordnet ist, um der Fluidzufuhrleitung (3) Spülungsfluid zuzuführen, wobei der Behälter auf einer vorbestimmten Höhe über der Endspitze (4) angeordnet ist, um einen gewünschten Fluidfluss von der Endspitze (4) durch Schwerkraft einem Auge (9) eines Patienten zuzuführen.

## Revendications

1. Dispositif d'irrigation ophtalmique (1) comprenant :
un support (6) apte à être monté sur un microscope (7) ayant un corps (7a) et un objectif (8), le support (6) étant apte à être monté sur le corps (7a) de telle sorte que le support (6) et l'objectif (8) sont déplaçables conjointement ; et
un conduit d'alimentation en fluide (3) ayant une pointe d'extrémité (4) destinée à distribuer du fluide d'irrigation à l'oeil (9) d'un patient ;
le support (6) ayant des moyens de fixation (13 ; 23, 24) agencés pour recevoir le conduit d'alimentation en fluide (3) et pour fournir une relation fixe et prédéterminée de la pointe d'extrémité (4) au support (6) ;
et les dimensions du support (6), des moyens de fixation (13 ; 23, 24) et du conduit d'alimentation en fluide (3) étant telles que la pointe d'extrémité (4), en utilisation,
est dans une position prédéterminée par rapport à l'objectif (8) sans qu'un quelconque réglage ne soit nécessaire, la position prédéterminée étant celle depuis laquelle, en utilisation, avec le conduit d'alimentation en fluide (3) en communication avec un contenant (2) de fluide d'irrigation à une hauteur prédéfinie, du fluide d'irrigation tombe au centre du champ de vision du microscope (7) lorsqu'un objet en observation à ce point est au foyer.

2. Dispositif selon la revendication 1, dans lequel le conduit d'alimentation en fluide (3) comprend un tube souple d'alimentation.

3. Dispositif selon la revendication 2, dans lequel le conduit d'alimentation en fluide (3) comprend une unité canule/connecteur (26, 13), la canule (26) étant agencée pour fournir la pointe d'extrémité (4) et le connecteur (13) étant agencé pour recevoir et communiquer avec le tube d'alimentation en fluide d'irrigation (3).

4. Dispositif selon l'une quelconque des revendications précédentes, dans lequel le conduit d'alimentation en fluide (3) est fixé au support (6) par une unité connecteur (23).

5. Dispositif selon la revendication 4, dans lequel le conduit d'alimentation en fluide (3) est fixé à l'unité connecteur (23) par une attache libérable (24).

6. Dispositif selon l'une des revendications 4 ou 5, dans lequel l'unité connecteur (23) comprend une attache à ressort (28) qui se positionne dans une cavité (27).

7. Dispositif selon l'une des revendications 4 ou 5, dans lequel l'unité connecteur (23) comprend un barreau (19) qui se positionne dans une fente (25).

8. Dispositif selon l'une quelconque des revendications précédentes, dans lequel le support (6) est rétractable et pend librement lorsqu'il se trouve dans la position étendue.

9. Microscope ophtalmique (7) comprenant un dispositif (1) selon l'une quelconque des revendications 1 à 8.

10. Microscope selon la revendication 9, comprenant un contenant (2) de fluide d'irrigation agencé pour alimenter en fluide d'irrigation le conduit d'alimentation en fluide (3), le contenant étant agencé à une hauteur prédéterminée au-dessus de la pointe d'extrémité (4) pour alimenter par gravité un écoulement désiré de fluide de la pointe d'extrémité (4) à l'oeil d'un patient (9).
